# EUROPEAN PATENT SPECIFICATION

(11) **EP 1 289 478 B1**
(45) Date of publication and mention of the grant of the patent: **08.09.2004**
(21) Application number: 01941007.5
(22) Date of filing: 06.06.2001
(51) Int. Cl.: A61K 7/06

(54) **COSMETIC COMPOSITIONS FOR THE CARE OF SCALP AND HAIR**
KOSMETISCHE ZUSAMMENSETZUNGEN ZUR BEHANDLUNG UND PFLEGE DER HAARE UND DER KOPFHAUT
COMPOSITIONS COSMETIQUES DESTINEES AU SOIN DU CUIR CHEVELU ET DES CHEVEUX

(30) Priority: 09.06.2000 IT MI000130
(43) Date of publication of application: 12.03.2003
(73) Proprietor: FARMAKA S.r.l., 22070 Grandate (Como) (IT)
(72) Inventor: CASERO, Alessandro, I-22100 Como (IT)
(74) Representative: Aimi, Luciano
(86) International application number: PCT/IT2001/000289
(87) International publication number: WO 2001/093817

(56) References cited:
- WO-A-99/12519
- US-A- 5 922 359

## Description

The present invention relates to cosmetic compositions for the care of hair, and in particular to a composition allowing to remove the deposits of impurities often arising on scalp and hair.

It is known that deposits of impurities consisting of variously originating substance such as furfuraceous scales, degradation products of sebum and sweat, dust and various inorganic substances, such as calcium, deriving from water used in normal cleaning, may grow on hair and scalp. In particular the last named inorganic item favors the aggregation of all these substances, making thus the resulting deposits specially hard and sticking to hair and scalp. In addition, just because of the presence of calcium said deposits are insoluble in water and cannot be disgregated and cleared away by means of usual detergents.

The production of such deposits results immediately in a worsening of the aesthetic look of hair that becomes shortly rough and opaque. A more serious consequence resides in that said deposits clog the pores and hair follicles, thus preventing the cutaneous respiration and causing the weakening and in the long run the loss of hair.

It is therefore an object of the present invention to provide a product able to remove said deposits from hair and scalp thus eliminating the cause of their weakening. Said object is attained by means of a cosmetic composition in form of a creamy emulsion whose principal features are emollient substances, an abrasive granular substance, a calcium chelating agent, a keratolytic agent and an antibacterial agent.
- A first advantage of the cosmetic composition according to the present invention resides in that, thanks to the calcium chelating agents and granular substances therein contained, it can be used in eliminating one of the reasons of hair loss and therefore in fighting the baldness. According to a particular embodiment, the composition in conformity with the present invention comprises also a compound having a keratolytic effect and able to favor the disintegrating action carried out on the noxious deposits.
- Another advantage of the cosmetic composition according to the present invention resides in that, by aiding the separation and removal of dead cells, it has also a marked anti-dandruff action.
- A further advantage of the cosmetic composition according to the present invention resides in that it contains also some emollient substances which contribute to improve the aesthetic look of hair by making it bulky, strong and glossy, and assist in its combing.
- A particular advantage of the composition according to the present invention resides in that, thanks to the antibacterial compounds contained therein, it prevents the degradation of sebum and sweat and avoid the formation of said deposits.

Further advantages and features of the cosmetic compositions according to the present invention will appear to those skilled in the art from the following detailed description of an embodiment thereof.

The cosmetic composition according to the present invention consists of a creamy emulsion of conventional type further comprising an abrasive granular substance, a calcium chelating agent, a keratolytic compound and an antibacterial agent.

Such a granular substance is adapted to allow for removal of noxious deposits formed on hair and scalp by applying through the fingers on it a delicate massage. Granular substances usable in the composition according to the present invention are all the substances conventionally used in products for the cleaning of skin, for example microcrystalline silica, calcium carbonate, tin oxide, magnesium stearate, synthetic resins, microcrystalline cellulose, Gransilk™ and the like.

The chelating agent has otherwise the task to complex the calcium ions by separating them from said noxious deposits so as to further aiding their disintegration. Various chelating agents known in the art may be added in the composition according to the present invention, such as for instance ethylenediaminetetraacetic acid, sodium heptagluconate, etidronic acid. Preferably, ethylenediaminetetraacetic acid or its salt is used; its amount by weight is preferably between 0,01% and 0,1% of the composition according to the present invention.

The composition in conformity with the present invention comprises also a compound having a keratolytic action that affects and dissolves the epidermis particles enclosed in said noxious deposits, thus making more effective the disgregating action on the latter. Such a keratolytic compound is preferably salicylic acid, and its amount by weight in the composition according to the present invention is between 0,001% and 0,05%.

The composition in conformity with this application comprises also an antibacterial compound having the task to prevent the decomposition of sebum and sweat, so as to oppose the formation of noxious deposits. Said antibacterial compound is preferably chlorexidine, and its amount by weight in the composition is between 0,01% and 1%.

Thanks to this last ingredient, the composition according to said embodiment of the invention is able to prevent the formation of noxious deposits on scalp and hair, which formation, as stated above, is linked to the decomposing bacterial activity on some organic residues. The composition can therefore be also used to preserve the proper bacterial flora of the scalp, which is essential to keep safe the good health of hair too.

A non limiting example of a preparation according to the invention is as follows.

### EXAMPLE

In a founding furnace the following ingredients are heated to 65°C until a clear mixture is obtained:

| | | |
|---|---|---|
| Cetylstearyl alcohol | kg | 56 |
| Cetyl alcohol | " | 30 |
| Cetylstearyl ether polyethoxylate | " | 20 |
| Paraffin oil | " | 20 |
| Lanolin | " | 10 |
| Laurylmethicone | " | 20 |
| Methylparaben | " | 2,0. |

In an emulgator are then heated to 70°C:

| | | |
|---|---|---|
| Demineralised water | q.s. to 1000 kg | |
| EDTA | kg | 2 |
| Sodium heptagluconate | " | 2 |
| Etidronic acid | " | 1,5 |
| Salicylic acid | " | 0,2 |

Both phases thus obtained are then homogenized for 15-20 minutes and to the resulting emulsion there are subsequently added:

| | | |
|---|---|---|
| Gransilk^{TM} | kg | 3 |
| Maize starch | " | 20 |
| Microcrystalline cellulose | " | 20 |

A de-aeration and homogenization step is then effected until a homogeneous mass is obtained. The mass is afterwards allowed to cool down to 40°C, and under swift agitation are added:

| | | |
|---|---|---|
| Cetyltrimethylammonium chloride | kg | 30 |
| Chlorexidine bichlorhydrate | " | 1 |
| Perfume | q.s. | |

A gentle agitation under vacuum is continued, and cooling down to the ambient temperature is carried out by setting the pH at 4-4,5 with possible regulation by means of lactic or citric acid.

The cosmetic composition thus obtained is a creamy emulsion to be used as a "mask" by gently massaging it on wet hair before or after a shampoo, allowing it to act for 3-10 minutes and rinsing then with water.

## Claims

1. A cosmetic composition for removing deposits of impurities from scalp and hair, comprising emollient substances, an abrasive granular substance, a calcium chelating agent, a keratolytic compound and an antibacterial agent.

2. The cosmetic composition according to claim 1, **characterized in that** said granular substance is selected from the group formed by microcrystalline silica, calcium carbonate, zinc oxide, magnesium stearate, microcrystalline cellulose and synthetic resins; said calcium chelating agent is selected from the group comprising sodium heptagluconate, etidronic acid, and ethylenediaminotetraacetic acid; said keratolytic compound is salicylic acid; and said antibacterial agent is chlorhexidine.

3. The cosmetic composition according to claim 3, wherein the amount by weight of ethylenediaminotetraacetic acid is comprised between 0.01% and 0.1%, salicylic acid between 0.001% and 0.05%, chlorhexidine between 0.01% and 1%.

## Patentansprüche

1. Kosmetische Zusammensetzung zur Entfernung von Fremdstoffen aus Kopfhaut und Haare, die erweichende Stoffe, einen körnigen Schleifstoff, ein Kalziumfangmittel, eine keratolytische Verbindung und ein antibakterielles Mittel enthält.

2. Kosmetische Zusammensetzung gemäss Anspruch 1, **dadurch gekennzeichnet, dass** der genannte körniger Stoff aus der von mikrokristalliner Kieselsäure, Kalziumkarbonat, Zinkoxyd, Magnesiumstearat, mikrokristalliner Zellulose und synthetischer Harze bestehenden Gruppe ausgewählt wird; das genannte Kalziumfangmittel aus der von Natriumheptaglukonat, Etidronsäure und Äthylendiaminotetraessigsäure bestehenden Gruppe ausgewählt wird; die genannte keratolytische Verbindung Salizylsäure ist; und das genannte antibakterielle Mittel Chlorhexidin ist.

3. Kosmetische Zusammensetzung gemäss Anspruch 2, wobei die Gewichtsmenge von Äthylendiaminotetraessigsäure zwischen 0,01% und 0,1%, von Salizylsäure zwischen 0,001% und 0,05%, und von Chlorhexidin zwischen 0,01% und 1% einbegriffen ist.

## Revendications

1. Composition cosmétique pour enlever les dépôts d'impuretés du cuir chevelu et des cheveux, comprenant des substances émollientes, une substance abrasive granulaire, un agent chélatant le calcium, un composé kératolytique et un agent antibactérien.

2. Composition cosmétique selon la revendication 1, **caractérisée en ce que** ladite substance granulaire est choisie dans le group formé de silice microcristalline, carbonate de calcium, oxyde de zinc, stéarate de magnésium, cellulose microcristalline et résines synthétiques; le dit agent chélatant le calcium est choisi dans le group comprenant le heptagluconate de sodium, l'acide étidronique et l'acide éthylenediaminotetraacétique; ledit composé kératolytique est l'acide salicylique; et ledit agent antibactérien est la chlorhexidine.

3. Composition cosmétique selon la revendication 2, dans laquelle la quantité en poids d'acide éthylenediaminotetraacétique est comprise entre 0,01% et 0,1%, d'acide salicylique entre 0,001 et 0,05%, de chlorhexidine entre 0,01% et 1%.
